# EUROPÄISCHE PATENTSCHRIFT

(11) **EP 0 502 524 B1**
(45) Veröffentlichungstag und Bekanntmachung des Hinweises auf die Patenterteilung: **15.05.1996**
(21) Anmeldenummer: 92103779.2
(22) Anmeldetag: 05.03.1992
(51) Int. Cl.: C12P 17/00, C12N 15/00

(54) **Mikrobiologisches Verfahren zur terminalen Hydroxylierung von Ethylgruppen an aromatischen 5- oder 6-Ring-Heterocyclen**
Microbiological process for terminal hydroxylation of ethyl-groups on aromatic 5- or 6-membered heterocycles
Procédé microbiologique pour l'hydroxylation terminale de groupes éthyliques sur des hétérocycles aromatiques à 5 ou 6 atomes

(30) Priorität: 07.03.1991 CH 696/91
(43) Veröffentlichungstag der Anmeldung: 09.09.1992
(73) Patentinhaber: LONZA AG, CH-3945 Gampel/Wallis (CH)
(72) Erfinder: Kiener, Andreas, Dr., Visp (Kanton Wallis) (CH); Zimmermann, Thomas, Dr., Naters (Kanton Wallis) (CH)
(74) Vertreter: Weinhold, Peter, Dr.

(56) Entgegenhaltungen:
- EP-A- 0 277 674
- AT-B- 374 495

## Beschreibung

Die Erfindung betrifft ein neues mikrobiologisches Verfahren zur terminalen Hydroxylierung von Ethylgruppen an aromatischen 5- oder 6-Ring-Heterocyclen sowie ein neues Hybridplasmid für das Verfahren.

2-Hydroxyethylderivate sind wichtige Zwischenprodukte zur Herstellung von pharmazeutischen Wirkstoffen, wie beispielsweise 2-(4-Pyridyl)ethanol zur Herstellung von Penicillinsäure-Derivaten.

Bekannt ist, dass die biochemische Oxidation von Alkanen in Mikroorganismen der Gattung Pseudomonas oleovorans in drei Schritten verläuft. Durch Einwirkung des Alkan-Hydroxylase-Komplexes (alkBA-Gene, die im folgenden als alkBFGH-Gene bezeichnet werden), entsteht zuerst der entsprechende Alkohol, der dann in zwei weiteren Schritten, katalysiert durch eine Alkoholdehydrogenase (alkC-Gene) und eine Aldehyddehydrogenase (chromosomal oder plasmidcodiert), zur Säure überführt wird. In diesem Stamm liegen die Gene, welche für die Enzyme der Oxidation verantwortlich sind, auf dem Plasmid OCT (Witholt et al., TIBTECH, Vol. 8, 1990, S.46-52).

Weiterhin ist aus der EP-A 277 674 ein mikrobiologisches Verfahren zur terminalen Hydroxylierung von apolaren aliphatischen Verbindungen mit 6 bis 12 C-Atomen,wie die Herstellung von 1-Octanol mittels Mikroorganismen der Gattung Pseudomonas oleovorans oder Pseudomonas putida, die gegen apolare Phasen resistent sind, bekannt.

Wie aus dem Ausführungsbeispiel hervorgeht, ist es zwingend, dass die Hydroxylierung dieser Verbindungen in einem aufwendigen Zwei-Phasen-System durchgeführt wird.

Mittels chemischen Verfahren sind 2-Hydroxyethylderivate von 5- oder 6-Ring-Heterocyclen ebenfalls schwer zugänglich.

Die Aufgabe der vorliegenden Erfindung bestand folglich darin, ein einfaches mikrobiologisches Verfahren zur terminalen Hydroxylierung von Ethylgruppen an aromatischen 5- oder 6-Ring-Heterocyclen zur Verfügung zu stellen.

Diese Aufgabe konnte auf überraschende Weise gemäss Patentanspruch 1 gelöst werden.

Erfindungsgemäss wird das Verfahren mit Mikroorganismen durchgeführt, die
a) die Gene von einem Pseudomonas OCT-Plasmid enthalten, die eine aktive Alkan-Monooxygenase bilden,
b) keine wirksame chromosomal- oder plasmidcodierte Alkoholdehydrogenase bilden
und damit befähigt sind,Ethylgruppen von aromatischen 5- oder 6-Ring-Heterocyclen zum entsprechenden Hydroxyethylderivat zu hydroxylieren, wobei der Heterocyclus als Substrat für die Umsetzung dient und das Hydroxyethylderivat nicht weiter metabolisiert wird.

Zweckmässig enthalten die Mikroorganismen die Gene alkBFGH von einem Pseudomonas OCT-Plasmid, die für den Alkan-Hydroxylase-Komplex codieren. Dabei sind zweckmässig die alkC-Gene oder andere Gene, die für aktive Alkoholdehydrogenasen codieren, entfernt oder inaktiviert.

### Quelle der Alkan-Monooxygenase-Gene

Als Quelle der Alkan-Monooxygenase-Gene dient das OCT-Plasmid von beispielsweise Pseudomonas oleovorans oder anderen Alkanverwertenden Mikroorganismen.

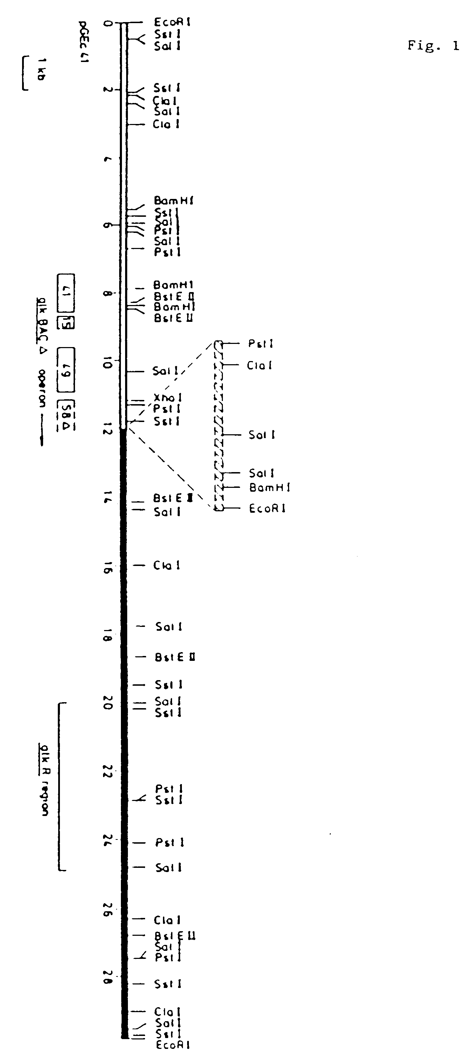

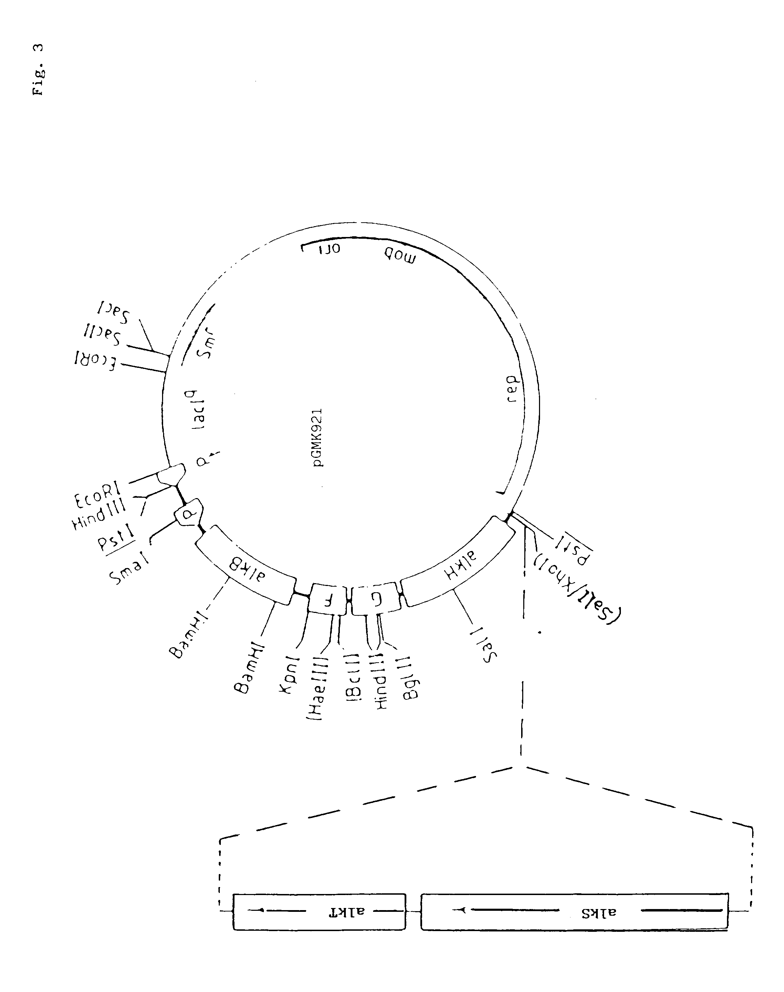

Ebenso geeignet sind konstruierte Hybridplasmide mit den Alkan-Monooxygenase-Genen, die in anderen Mikroorganismen enthalten sind, wie beispielsweise in E.coli oder in Pseudomonas putida. Als solches Hybridplasmid kann beispielsweise das Plasmid pGEc41, Fig. 1, hinterlegt entweder in E.coli DH1 (CBS 102-87) oder hinterlegt in E.coli DH1 (DSM 6726), dienen. Das neu konstruierte Hybridplasmid pGMK921, Fig. 3, hinterlegt entweder in Pseudomonas putida PpS81 (DSM 6776) oder in Pseudomonas putida GPO12 (DSM 6775), kann ebenfalls als Quelle für die Alkan-Monooxygenase-Gene dienen.

Die genetische Information, die für die Alkan-Monooxygenase codiert, kann in der Weise erhalten werden, indem man a) entweder die OCT-Plasmid-DNA oder die Hybridplasmid-DNA aus Mikroorganismen isoliert, dann b) diese DNA zur Isolation des Alkan-Monooxygenase-Gens verdaut und diese spezifische Gensequenz dann c) in einen Expressionsvektor einführt, wobei dadurch ein Hybridplasmid d) entsteht. Dieses Hybridplasmid kann dann in einen für das Verfahren geeigneten Mikroorganismus e) (Wirtsstamm) mittels Transformation f) eingebracht werden. Dieser transformierte Wirtsstamm e) bildet dann den Produktionsstamm g) für das erfindungsgemässe Verfahren.

### a) Isolation der OCT-Plasmid-DNA (Hybridplasmid-DNA)

Sowohl die OCT-Plasmid-DNA als auch die Hybridplasmid-DNA kann nach fachmännisch üblichen Methoden erhalten werden. Dabei wird beispielsweise der Mikroorganismus vollständig lysiert, wobei die gewünschte Plasmid-DNA dann mittels Dichtegradientenzentrifugation erhalten werden kann. Hierzu kann beispielsweise auf das Lehrbuch "Current Protocols in Molecular Biology", Asubel et al., Eds.; J. Wiley, New York, 1989, Abschnitt 2 verwiesen werden.

### b) Spaltung der Plasmid-DNA mit Restriktionsenzymen

Nach der Isolation der Plasmid-DNA kann diese nach fachmännisch üblichen Methoden mit Restriktionsenzymen so gespalten werden, dass die DNA keine plasmidcodierte Alkoholdehydrogenase bildet.

Der erhaltene DNA-Abschnitt (alkBFGH), der für die aktive Alkan-Monooxygenase codiert, kann dann beispielsweise durch Agarose-Gel-Elektrophorese isoliert werden.

### c) Ligation des DNA-Abschnitts in Expressionsvektoren

Der so erhaltene Genabschnitt alkBFGH kann mittels üblichen molekularbiologischen Techniken mit einer zuvor gleichermassen geschnittenen Expressionsvektor-DNA ligiert werden, wie beispielsweise beschrieben in J. Biol. Chem. 1989, 264, S.5442-5451 (M. Kok et al.).

Expressionsvektoren enthalten üblicherweise einen geeigneten, meist regulierbaren Promotor. Hinter diesem Promotor liegen günstigerweise in Transkriptionsrichtung eine oder mehrere singuläre Schnittstellen für Restriktionsenzyme. In diese Schnittstellen wird dann üblicherweise der gewünschte Genabschnitt inseriert, an dessen Expression man interessiert ist.

Die Transkription der alkBFGH-Gene kann sowohl durch den alk-Promotor unter natürlicher Regulation (pGEc41) oder sowohl durch den Promotor tacI als den alk-Promotor unter natürlichen Regulation initiiert werden (pGMK921).

Grundsätzlich sind alle Expressionsvektoren geeignet, welche den Genabschnitt alkBFGH in dem gewählten Wirt zu replizieren und zu exprimieren vermögen.

### d) Hybridplasmide

Die zweckmässig so entstandenen Hybridplasmide weisen insbesondere ein breites Wirtsspektrum auf und können dementsprechend in Wirtsstämmen mit hoher Substrat- und Edukttoleranz eingesetzt werden.

Insbesondere wird das Hybridplasmid pGEc41 angewendet, welches in E.coli DH1 sowohl am 15. Januar 1987 beim "Centralbureau voor Schimmelcultures at Baarm" in den Niederlanden mit der Nummer CBS 102-87 als auch am 30. September 1991 bei der Deutschen Sammlung für Mikroorganismen und Zellkulturen GmbH (DSM), Mascheroderweg 1b, D-3300 Braunschweig, mit der Nummer DSM 6726, hinterlegt wurde.

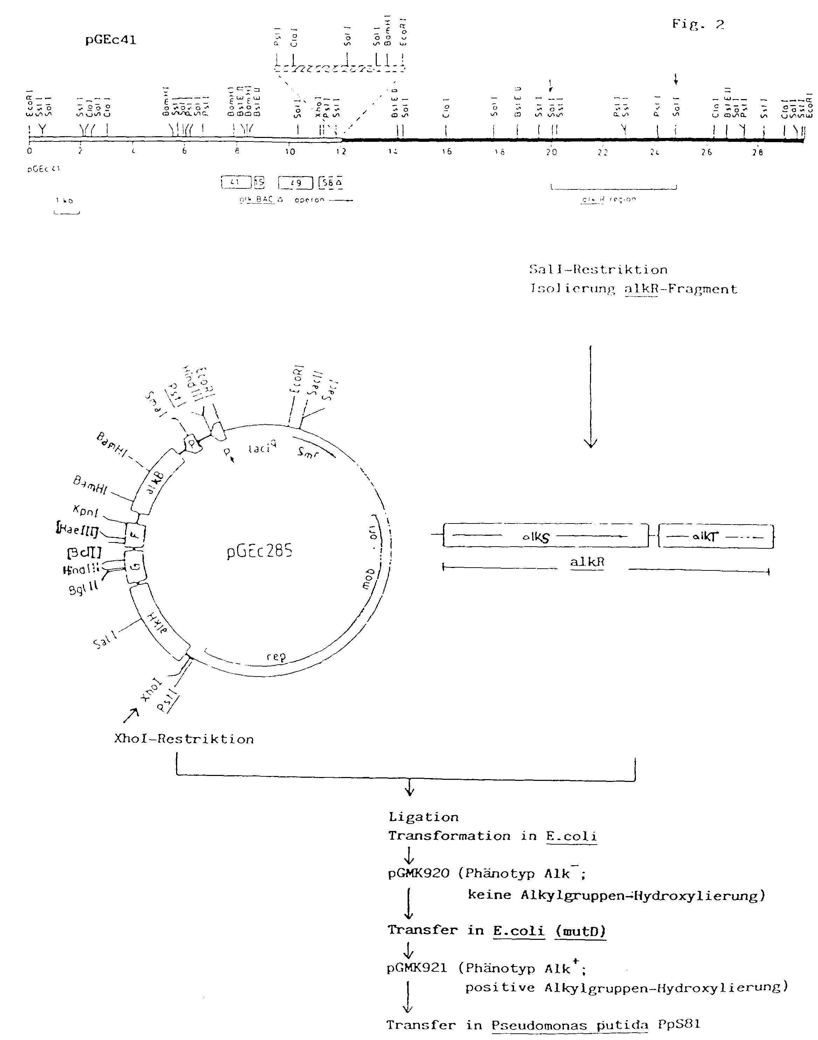

Ein weiteres bevorzugt geeignetes Hybridplasmid ist das Hybridplasmid pGMK921, Fig. 3, welches sowohl in Pseudomonas putida PpS81 (DSM 6776) als auch im Pseudomonas putida GP012 (DSM 6775) am 6. November 1991 bei der DSM hinterlegt wurde. Dieses erfindungsgemässe Hybridplasmid stellt eine Weiterentwicklung des bereits bekannten Hybridplasmids pGEc285 (J. Biol. Chem., 1989, 264, S.5442-5451) dar. Die Konstruktion von Hybridplasmid pGMK921 ist in Fig. 2 schematisch dargestellt.

### e) Wirtsstämme

Die so erhaltenen Hybridplasmide können in eine Vielzahl von Wirtsstämmen eingesetzt werden.

Zweckmässig werden Wirtsstämme mit hoher Substrat- und Edukttoleranz eingesetzt, wie z.B. Mikroorganismen der Gattung Pseudomonas, Acinetobacter, Rhizobium, Agrobacterium oder Escherichia.

Vorzugsweise werden als Wirtsstämme E.coli DH1, Pseudomonas putida PpS81 oder Pseudomonas putida GP012 angewendet.

### f. Transformation

Die Transformation der Wirtszellen mit den Hybridplasmiden, woraus die erfindungsgemässen Produktionsstämme resultieren, ist ebenfalls an sich bekannt und eingehend in dem zuvor angegebenen Lehrbuch beschrieben.

### g. Produktionsstämme

Als Produktionsstämme können alle Wirtsstämme dienen, die mit den Hybridplasmiden, die den Genabschnitt alkBFGH enthalten, transformiert sind.

Die natürliche Regulation des alk-Operons kann beibehalten oder entkoppelt sein.

Insbesondere dienen als Produktionsstämme E.coli DH1, transformiert mit dem Hybridplasmid pGEc41 (CBS 102-87 oder DSM 6726), Pseudomonas putida PpS81 (DSM 6776) oder Pseudomonas putida GP012 (DSM 6775), beide transformiert mit dem Hybridplasmid pGMK921, oder wirksame Mutanten von diesen Stämmen.

In diesen Produktionsstämmen ist die natürliche Regulation des alk-Operons (alkR = alkST) beibehalten.

### Selektion der transformierten Mikroortanismen (Produktionsstämme)

Die Isolierung (Selektion) der transformierten Wirtszellen erfolgt vorteilhaft aus einem selektiven Nährmedium, dem das Biocid zugesetzt wird, gegen welches das im Hybridplasmid enthaltene Markierungs-Gen Resistenz verleiht. Wenn, wie bevorzugt, das Hybridplasmid pGEc41 das Tetracyclin-Resistenz-Gen enthält, wird demgemäss dem Nährmedium Tetracyclin zugesetzt. Im Falle des Hybridplasmids pGMK921, das das Streptomycin-Resistenz-Gen enthält, wird demgemäss dem Nährmedium Streptomycin zugesetzt. Zellen, die dieses Hybridplasmid nicht enthalten, werden in einem solchen Medium am Wachstum gehemmt.

### Fermentationsverfahren

Erfindungsgemäss können als Produktionsstämme alle angewendet werden, die mit einem Hybridplasmid, das den Genabschnitt alkBFGH enthält, transformiert sind, wie beispielsweise transformierte Mikroorganismen der Gattung Pseudomonas, Acinetobacter, Rhizobium, Agrobacterium oder Escherichia.

Das erfindungsgemässe Fermentationsverfahren wird mit dem Produktionsstamm E.coli DH1, transformiert mit dem Hybridplasmid pGEc41, nachstehend erläutert.

Als Substrate können für die Umsetzung ethylierte aromatische 5- oder 6-Ring-Heterocyclen dienen, die ein oder mehrere Heteroatome aus der Reihe Sauerstoff, Stickstoff, Schwefel enthalten.

Als aromatische 5-Ring-Heterocyclen können beispielsweise ethylierte Thiophen-, Furan-, Pyrrol-, Thiazol-, Pyrazol-oder Imidazolderivate dienen.

Geeignete aromatische 6-Ring-Heterocyclen sind beispielsweise ethylierte Pyridin-, Pyrimidin-, Pyrazin- oder Pyridazinderivate.

Insbesondere dienen als aromatische ethylierte 6-Ring-Heterocyclen Derivate aus der Verbindungsklasse der Pyrazine oder Pyridine.

Bei E.coli DH1, enthaltend pGEc41, als Produktionsstamm können die zur Hydroxylierung verantwortlichen Enzyme des alk-Operons mit Verbindungen induziert werden, die bereits bei Grund et al., J.Bacteriol., 123, S.546-556, 1975, beschrieben sind. Dazu gehören Verbindungen, die beispielsweise für Pseudomonas oleovorans als Kohlenstoff- und Energiequelle dienen, wie beispielsweise Alkane, Alkanole oder alkylierte cyclische Verbindungen.

Als Alkane können beispielsweise Octan, Dodecan oder Hexan angewendet werden.

Als Alkanole können beispielsweise Octanol, Dodecanol oder Hexanol angewendet werden

Als Vertreter der alkylierten cyclischen Verbindungen kann beispielsweise Ethylbenzol Verwendung finden.

Verbindungen, die beispielsweise Pseudomonas oleovorans nicht als Kohlenstoff- und Energiequelle dienen, die aber die Gene des alk-Operons trotzdem induzieren, sind beispielsweise Dicyclopropylketon, Dicyclopropylmethanol oder Diethoxyethan. Vorzugsweise wird die Enzyminduktion bei E.coli DH1, enthaltend pGEc41, mit Dicyclopropylketon durchgeführt.

Die Umsetzung des Substrates kann sowohl in Anwesenheit des Enzyminduktors als auch in Abwesenheit des Enzyminduktors erfolgen.

Für das Wachstum des genannten Produktionsstammes können alle fachmännisch üblichen Kohlenstoff- und Energiequellen angewendet werden, wie beispielsweise Natriumsuccinat.

Zweckmässig wird zur Stabilisierung des Plasmides pGEc41 vor der Umsetzung des Substrates (bei der Zellanzucht) Tetracyclin dem Kultivierungs-Medium zugegeben.

Als Kultivierungs-Medium dienen die in der Fachwelt üblichen Medien, wie beispielsweise ein Komplexmedium ("Nutrient Broth No. 2", Oxoid Ltd., England) oder ein Mineralsalzmedium, wie beispielsweise beschrieben in Kulla et al., Arch.Microbiol. 135, 1983, S.1-7.

Vor der Substratzugabe werden die Zellen auf übliche Weise gezüchtet, und anschliessend wird die Umsetzung des Substrates zweckmässig bei einer optischen Dichte von 1 bis 200 bei 650 nm in Kulturmedium durchgeführt, vorzugsweise bei einer optischen Dichte von 5 bis 100 bei 650 nm.

Die Umsetzung kann entweder unter einmaliger oder kontinuierlicher Substratzugabe erfolgen, so dass die Substratkonzentration im Kulturmedium 20% (w/v) nicht übersteigt.

Vorzugsweise erfolgt die Substratzugabe so, dass die Substratkonzentration im Kulturmedium 5% (w/v), insbesondere 1% (w/v), nicht übersteigt.

Die Umsetzung wird zweckmässig in einem pH-Bereich von 4 bis 11, vorzugsweise von 6 bis 10, durchgeführt.

Die Umsetzung wird üblicherweise bei einer Temperatur von 15 bis 50°C, vorzugsweise bei einer Temperatur von 25 bis 40°C, durchgeführt.

Zweckmässig wird die Umsetzung über eine Zeit von 1 Stunde bis zu mehreren Tagen, vorzugsweise kontinuierlich über mehrere Tage, durchgeführt.

Nach der Umsetzung können die entsprechenden 2-Hydroxyethyl-Derivate auf bekannte Art und Weise, wie beispielsweise durch Extraktion, isoliert werden.

### Beispiel 1

E.coli DH1 (pGEc41), CBS 102-87, wurde in einem Mineralsalzmedium (Kulla et al., Arch. Microbiol. 135, 1983, S.1-7) mit 0,6% (w/v) Natriumsuccinat als einziger Kohlenstoff- und Energiequelle angezogen.

Zur Stabilisierung des Plasmids wurden 25 mg/l Tetracyclin dem Medium zugegeben.

Als Enzyminduktor diente Dicyclopropylketon in einer Konzentration von 1 mmol/l.

Zur Zellsuspension (100 ml) mit einer optischen Dichte von 10 bei 650 nm wurde 1 mmol 5-Ethyl-2-methylpyridin, was einer Konzentration von 0,12% (w/v) entsprach, hinzugegeben, und die Zellsuspension wurde für weitere 16 h bei einer Temperatur von 30°C und bei einem pH-Wert von 7 inkubiert.

Unter diesen Bedingungen wurde 1 mmol (121 mg/100 ml) 5-Ethyl-2-methylpyridin zu 0,8 mmol 5-(2-Hydroxyethyl)-2-methylpyridin, entsprechend einer Ausbeute von 80%, bezogen auf eingesetztes 5-Ethyl-2-methyl-pyridin, umgesetzt.

Ensprechend wurde die Umsetzung mit E.coli DH1 (pGEc41), DSM 6726,durchgeführt.

Die Beispiele 2 und 3 wurden entsprechend zu Beispiel 1 durchgeführt und sind in Tabelle 1 zusammengefasst.

### Beispiel 4

### I Konstruktion des Hybridplasmides pGMK921 (Fig. 2)

Die Konstruktion von pGMK921 ist eine regulative Weiterentwicklung von pGEc285 (siehe Fig. 2). pGEc285 ist beschrieben in J. Biol. Chem. 264 (1989), S.5442-5451) (M. Kok et al.). Dieses Plasmid ist ein Derivat des "multicopy number broad host range" Vektors pMMB24 (Gene 26 (1983), S.273-282) und enthält ein PstI-DNA-Fragment vom OCT-Plasmid respektive vom pGEc41 (J. Biol. Chem. 262 (1987), S.17712-17718), welches für die cytoplasmatische Membrankomponente der Alkanhydroxylase (AlkB), zwei Rubretoxine (AlkF, AlkG) und für eine Aldehyddehydrogenase (AlkH) codiert. Transkription von alkBFGH (= alkBA) kann sowohl durch den alk-Promotor als auch durch den tacI-Promotor initiiert werden. Für die Genexpression über den alk-Promotor unter natürlicher Regulation wurden in pGEc285 die alkST-Cistrons (= alkR) vom OCT-Plasmid beziehungsweise von pGEc41 hineinligiert.

pGEc41 wurde mit SalI (4 units pro µg Plasmid-DNA) geschnitten, und die DNA-Fragmente wurden über präparative Agarose-Gelelektrophorese (0,6% (w/v) Agarose in TBE-Puffer (0,09 M Tris-Borat, 2,5 mM Na₂EDTA, pH 8,3, Ethidiumbromid (100 µg/100 ml)) aufgetrennt. Das 2,5 kb grosse SalI-Fragment, welches für alkST codiert, wurde direkt aus dem Agarosegel über DEAE-Cellulose-Membran isoliert. Die aufgelaufene DNA wurde von der Membran mittels 0,5 ml Elutionspuffer (20 mM Tris-HCl, pH 7,5, 1 mM Na₂EDTA, 1,5 M NaCl) während 1 h bei 65°C abgewaschen. Das Ethidiumbromid wurde mit H₂O-gesättigtem n-Butanol aus der DNA-Probe extrahiert und die DNA anschliessend mit Isopropanol präzipitiert. Der getrocknete Niederschlag dieses Fragmentpräparats, im folgenden als Präparat (a) bezeichnet, wurde in 0,01 M Tris-HCl-Puffer, pH 8,0, 0,001 M Na₂EDTA aufgenommen.

pGEc285 wurde mit XhoI (4 units pro µg Plasmid DNA) geschnitten und ebenfalls präzipitiert. Das getrocknete Plasmidpräparat, im folgenden als Präparat (b) bezeichnet, wurde im gleichen Puffer aufgenommen.

Beide Präparate, (a) und (b), 0,1-4 µg DNA in 20 µl, wurden einer Klenow-Reaktion entsprechend Vorschrift (Maniatis et al., Molecular Cloning 1989, Cold Spring Harbor Laboratory Press, Abschnitt 5.40-5.43) unterzogen. Klenow-Puffer: 50 mM Tris-HCl, pH 7,5, 10 mM MgCl₂, 1 mM Dithiothreitol, 50 µg/ml Rinderserumalbumin.

Zum Reaktionsgemisch wurde 1 µl 0,5 mM von jedem dNTP (Desoxynukleotidtriphosphat) zugegeben. Nach Zugabe von 1-5 units Klenow-Polymerase wurde bei 30°C für 15 min inkubiert. Anschliessend wurde die Reaktion durch Erhitzen auf 75°C für 10 min abgebrochen.

Zur Ligation wurden die "blunt-ends" (Glatte DNA-Enden ohne überstehende Einzelstrangbereiche) Präparate (a) und (b) mit Isopropanol präzipitiert und in 100 µl Ligationspuffer (20 mM Tris-HCl, pH 7,2, 10 mM DTT, 10 mM MgCl₂, 0,6 mM ATP) aufgenommen. Die Ligation erfolge nach Zugabe von T4-DNA-Ligase (1 unit/µg DNA) über Nacht bei 15°C.

Transformiert wurde in E.coli unter Selektion auf Komplexmedium mit 30 mg Streptomycin pro Liter Nutrientagar. (Elektrotransformation nach Maniatis et al., Molecular Cloning 1989, Cold Spring Harbor Laboratory Press, Abschnitt 1.75). Das Plasmid erhielt die Bezeichnung pGMK920 und vermittelte dem Wirtsstamm keine Fähigkeit zur Alkylgruppenhydroxylierung. Erst die Transformation in E.coli (mutD, Propagierung (Aussetzen von pGMK920) in "Mutator-Hintergrund") und anschliessender konjugativer Transfer (mit Helferplasmid pRK2013) in Pseudomonas putida , (Ps.Putida) PpS81, führte zu einem Alk⁺-Phänotyp des plasmidtragenden Rezipientenstamms (besitzt die Fähigkeit, Alkylgruppen zu hydroxylieren).

Zum konjugativen Transfer wurde E.coli mit dem alk-Hybridplasmid zusätzlich mit dem Helferplasmid pRK2013 elektrotransformiert (Maniatis et al.,ibid). Selektion erfolgte auf Komplexmedium (Nutrientagar) mit Streptomycin 30 mg/l (Hybridplasmid) und Kanamycin 25 mg/l (Helferplasmid).

Je 1 ml dieser Donorkultur und der Rezipientenkultur (Ps.putida PpS81) wurden mehrfach mit Nutrient Broth gewaschen, in je 50 µl Nutrient Broth aufgenommen, vereinigt und auf trockenem Nutrientagar über Nacht bei 30°C inkubiert. Die Zellen wurden in 0,9% NaCl-Lösung suspendiert und geeignete Verdünnungen (10⁻⁷) der 10⁹ Zellen/ml-Suspension wurden auf Selektivmedium (150 mg Streptomycin pro Liter) plattiert. Das nunmehr Alk⁺-Phänotyp vermittelnde Plasmid erhielt die Bezeichnung pGMK921.

### II Biotransformation

Der Produktionsstamm Ps.putida PpS81, enthaltend pGMK921, ist wegen der alcA-Mutation des Wirtes besonders geeignet, da die Alkanhydroxylase-Aktivität vor einem vollständig Alkoholdehydrogenase-freien Hintergrund exprimiert wird. Ps.putida PpS81 mit pGMK921 wurde auf Mineralsalzmedium (Arch. Microbiol. 135 (1983), S.1-7) mit 0,2% (w/v) Glukose angezogen. Zur Plasmidstabilisierung wurde Streptomycin (150 mg/l) zugegeben. Zur Induktion des natürlichen Promotors für die Alkan-Monooxygenase-Expression wurde 1 mM Dicyclopropylketon zugegeben.

Bei einer optischen Dichte von OD₆₅₀ₙₘ = 10 (Kulturen geringerer OD wurden konzentriert auf OD = 10) der Kultur wurden 0,1% (v/v) 5-Ethyl-2-methylpyrimidin zugegeben. Bei einer Temperatur von 30°C und einem neutralen pH-Wert (7,0) war dieses Substrat nach 6 h vollständig zu 5-(2-Hydroxyethyl)-2-methylpyrimidin umgesetzt.

### Beschreibung der Figuren

### Figur 1:

pGEc41 besteht aus dem Vektor pLAFR1 (Gene 16 (1986), S.289-296) und einem 30 kb grossen EcoRI-Fragment aus OCT-Plasmid-DNA,welches für einen Teil des alkBFGHC'-Operons (Deletion in alkC-Gen) codiert und dazu die alkR-loci trägt. Das gestrichelt wiedergegebene Fragment kennzeichnet die Deletion stromabwärts im alkBFGHC'-Operon.

Die Kästchen geben Grösse und Position der Proteine wieder, die Zahlen beziehen sich auf die Molekularmasse in Kilo-Dalton. Im pGEc41 ist die Orientierung der alkR-DNA-Sequenz und der alkBFGHC'-Sequenz identisch (J. Biol. Chem 262 (1987), S.17712-17718).

### Fig. 2.

Stellt das Konstruktionsschema zur Herstellung von pGMK921 dar.

### Figur 3:

pGMK920 ist hergeleitet von pGEc285 und pGEc41.

Die Trankriptionsrichtung von alkBFGH ist im Gegenuhrzeigersinn, die alkST (alkR) Cistrons werden im Uhrzeigersinn transkribiert.

Die Restriktionskarte von pGMK921, welches eine Alk⁺-Mutation enthält, ist identisch mit pGMK920.

Das Plasmid codiert für:
- Membrankomponente der Alkanhydroxylase (alkB)
- Rubredoxin (alkG)
- Rubredoxin-F (alkF)
- Aldehyddehydrogenase (alkH)
- Rubredoxinreduktase (alkS)
- positiver Regulator (alkS)

pGMK921 kann aus E.coli mittels Helferplasmid (pRK2013) in Pseudomonas konjugativ transferiert werden.

Selektion: Streptomycin 35 mg/l (E.coli)
Streptomycin 150 mg/l (Pseudomonas putida)

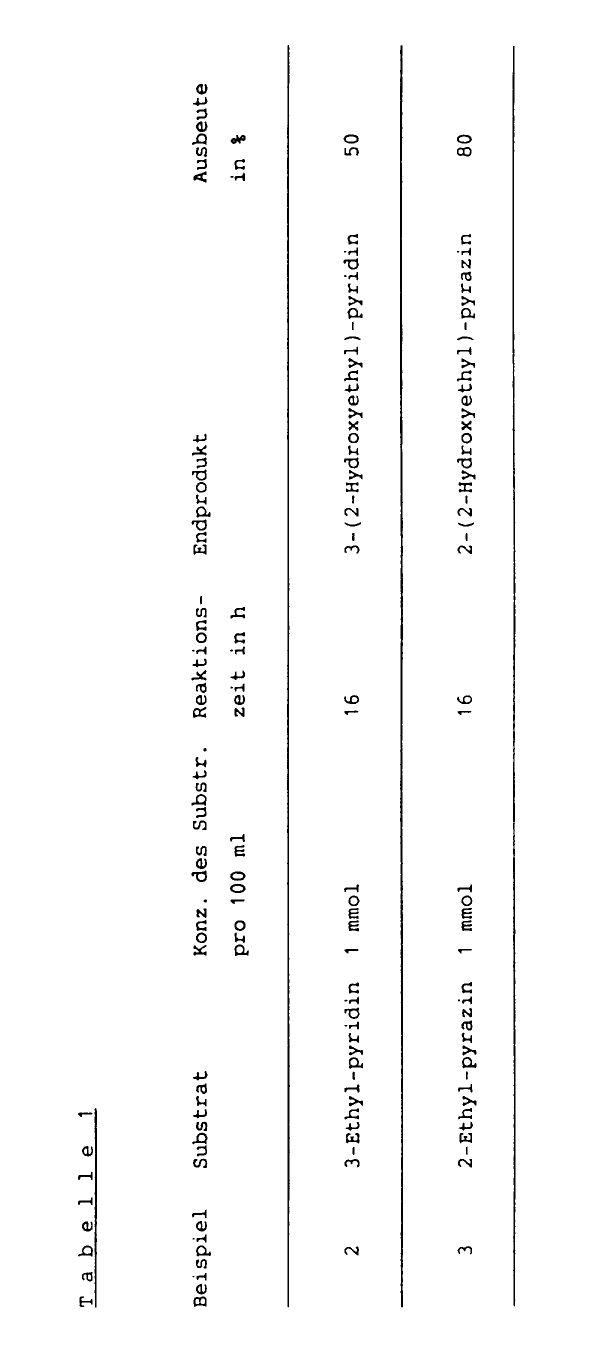

## Patentansprüche

1. Mikrobiologisches Verfahren zur terminalen Hydroxylierung von Ethylgruppen an aromatischen Heterocyclen, dadurch gekennzeichnet, dass man die Umsetzung mit Mikroorganismen durchführt, die
a) die Gene von einem Pseudomonas OCT-Plasmid enthalten, die eine aktive Alkan-Monooxygenase bilden,
b) keine wirksame chromosomal- oder plasmidcodierte Alkoholdehydrogenase bilden
und damit befähigt sind, Ethylgruppen von aromatischen 5- oder 6-Ring-Heterocyclen zum entsprechenden Hydroxyethylderivat zu hydroxylieren, wobei der Heterocyclus als Substrat für die Umsetzung dient und das Hydroxyethylderivat nicht weiter metabolisiert wird.

2. Verfahren nach Patentanspruch 1, dadurch gekennzeichnet, dass man die Umsetzung mit Mikroorganismen durchführt, die die Gene alkBA von einem Pseudomonas OCT-Plasmid enthalten.

3. Verfahren nach einem der Patentansprüche 1 und 2, dadurch gekennzeichnet, dass man die Umsetzung mit Mikroorganismen durchführt, die der Gattung Pseudomonas, Acinetobacter, Rhizobium, Agrobacterium oder Escherichia angehören.

4. Verfahren nach mindestens einem der Patentansprüche 1 bis 3, dadurch gekennzeichnet, dass man die Umsetzung entweder mit E.coli DH1 (CBS 102-87) oder mit E.coli DH1 (DSM 6726), beide transformiert mit dem Hybridplasmid pGEc41, oder einer wirksamen Mutante von diesen Stämmen durchführt.

5. Verfahren nach mindestens einem der Patentansprüche 1 bis 4, dadurch gekennzeichnet, dass man die Umsetzung entweder mit Pseudomonas putida PpS81 (DSM 6776) oder mit Pseudomonas putida GP012 (DSM 6775), beide transformiert mit dem Hybridplasmid pGMK921, oder mit einer wirksamen Mutante von diesen Stämmen durchführt.

6. Verfahren nach mindestens einem der Patentansprüche 1 bis 5, dadurch gekennzeichnet, dass man als Substrat einen ethylierten aromatischen 5- oder 6-Ring-Heterocyclus anwendet, der ein oder mehrere Heteroatome aus der Reihe Sauerstoff, Stickstoff, Schwefel enthält.

7. Verfahren nach mindestens einem der Patentansprüche 1 bis 6, dadurch gekennzeichnet, dass man als Substrat ein ethyliertes Pyrazin oder Pyridin anwendet.

8. Verfahren nach mindestens einem der Patentansprüche 1 bis 7, dadurch gekennzeichnet, dass die Enzyme des Mikroorganismus entweder mit Verbindungen induziert werden, die dem Mikroorganismus als Kohlenstoff- und Energiequelle dienen, oder mit Verbindungen, die dem Mikroorganismus nicht als Kohlenstoff- und Energiequelle dienen.

9. Verfahren nach mindestens einem der Patentansprüche 1 bis 8, dadurch gekennzeichnet, dass die Umsetzung unter einmaliger oder kontinuierlicher Substratzugabe erfolgt, so dass die Substratzugabe 20% (w/v) nicht übersteigt.

10. Verfahren nach mindestens einem der Patentansprüche 1 bis 9, dadurch gekennzeichnet, dass man die Umsetzung bei einem pH-Wert von 4 bis 11 durchführt.

11. Verfahren nach mindestens einem der Patentansprüche 1 bis 10, dadurch gekennzeichnet, dass man die Umsetzung bei einer Temperatur von 15 bis 50°C durchführt.

12. Hybridplasmid pGMK921, wie hinterlegt in Pseudomonas putida PpS81 (DSM 6776) und in Pseudomonas putida GP012 (DSM 6775).

## Claims

1. A microbiological process for the terminal hydroxylation of ethyl groups on aromatic heterocycles, characterized in that the reaction is performed with microorganisms which:
a) contain the genes of a Pseudomonas OCT plasmid, which
form an active alkane monooxygenase, b) form no active chromosomal or plasmid-coded alcohol dehydrogenase,
and are therefore capable of hydroxylating ethyl groups of aromatic 5- or 6-membered heterocycles to the corresponding hydroxyethyl derivative, wherein the heterocycle serves as substrate for the reaction, and the hydroxyethyl derivative is not further metabolized.

2. The process according to claim 1, characterized in that the reaction is performed with microorganisms, which contain the alkBA genes of a Pseudomonas OCT plasmid.

3. The process according to one of claims 1 and 2, characterized in that the reaction is performed with microorganisms, which belong to the genus Pseudomonas, Acinetobacter, Rhizobium, Aqrobacterium or Escherichia.

4. The process according to at least one of the claims 1 to 3, characterized in that the reaction is performed either with E. coli DH1 (CBS 102-87) or with E. coli DH1 (DSM 6726), each of which is transformed with hybrid plasmid pGEc41, or with an active mutant of these strains.

5. The process according to at least one of the claims 1 to 4, characterized in that the reaction is performed either with Pseudomonas putida PpS81 (DSM 6776) or with Pseudomonas putida GPO12 (DSM 6775), each of which is transformed with hybrid plasmid pGMK921, or with an active mutant of these strains.

6. The process according to at least one of the claims 1 to 5, characterized in that an ethylated aromatic 5- or 6-membered heterocycle which contains one or several heteroatoms from the group consisting of oxygen, nitrogen and sulfur, is used as the substrate.

7. The process according to at least one of the claims 1 to 6, characterized in that an ethylated pyrazine or an ethylated pyridine is used as the substrate.

8. The process according to at least one of the claims 1 to 7, characterized in that the enzymes of the microorganism are induced either with compounds, which are used by the microorganism as a carbon and energy source, or with compounds, which are not used by the microorganism as a carbon and energy source.

9. The process according to at least one of the claims 1 to 8, characterized in that the reaction takes place with single or continuous substrate addition, so that the substrate addition does not exceed 20 % (w/v).

10. The process according to at least one of the claims 1 to 9, characterized in that the reaction is performed at a pH value of 4 to 11.

11. The process according to at least one of the claims 1 to 10, characterized in that the reaction is performed at a temperature of from 15 °C to 50 °C.

12. Hybrid plasmid pGMK921, as deposited in Pseudomonas putida PpS81 (DSM 6776) and in Pseudomonas putida GPO12 (DSM 6775).

## Revendications

1. Procédé microbiologique pour l'hydroxylation terminale de groupes éthyle sur des hétérocycles aromatiques, caractérisé en ce que l'on effectue la réaction avec des microorganismes qui
a) contiennent les gènes d'un plasmide OCT de *Pseudomonas* qui forment une alcane monooxygénase active,
b) qui ne forment pas d'alcool déshydrogénase active chromosomique ou codée par un plasmide,
et qui sont ainsi capables d'hydroxyler des groupes éthyle d'hétérocycles aromatiques à 5 ou 6 chaînons pour donner le dérivé hydroxyéthylé correspondant, l'hétérocycle servant de substrat pour la réaction et le dérivé hydroxyéthylé n'étant pas métabolisé ultérieurement.

2. Procédé selon la revendication 1, caractérisé en ce que l'on effectue la réaction avec des microorganismes qui contiennent les gènes *alkBA* d'un plasmide OCT de *Pseudomonas.*

3. Procédé selon l'une des revendications 1 et 2, caractérisé en ce que l'on effectue la réaction avec des microorganismes qui appartiennent au genre *Pseudomonas, Acinetobacter, Rhizobium, Agrobacterium* ou *Escherichia.*

4. Procédé selon l'une au moins des revendications 1 à 3, caractérisé en ce que l'on effectue la réaction avec *E. coli* DH1 (CBS 102-87) ou avec *E. coli* DH1 (DSM 6726), transformées toutes les deux avec le plasmide hybride pGEc41, ou avec un mutant actif de ces souches.

5. Procédé selon l'une au moins des revendications 1 à 4, caractérisé en ce que l'on effectue la réaction avec *Pseudomonas putida* PpS81 (DSM 6776) ou avec *Pseudomonas putida* GP012 (DSM 6775), transformées toutes les deux avec le plasmide hybride pGMK921, ou avec un mutant actif de ces souches.

6. Procédé selon l'une au moins des revendications 1 à 5, caractérisé en ce que l'on utilise comme substrat un hétérocycle aromatique éthylé à 5 ou 6 chaînons qui contient un ou plusieurs hétéroatomes du groupe de l'oxygène, de l'azote et du soufre.

7. Procédé selon l'une au moins des revendications 1 à 6, caractérisé en ce que l'on utilise comme substrat une pyrazine ou pyridine éthylée.

8. Procédé selon l'une quelconque des revendications 1 à 7, caractérisé en ce que les enzymes du microorganisme sont induites avec des composés qui sont utilisés par le microorganisme comme source de carbone ou source d'énergie, ou avec des composés qui ne sont pas utilisés par le microorganisme comme source de carbone ou source d'énergie.

9. Procédé selon l'une au moins des revendications 1 à 8, caractérisé en ce que l'on effectue la réaction en ajoutant le substrat en une seule fois ou de manière continue, de façon que la quantité de substrat ajouté ne soit pas supérieure à 20 % (masse/volume).

10. Procédé selon l'une au moins des revendications 1 à 9, caractérisé en ce que la réaction s'effectue à un pH compris entre 4 et 11.

11. Procédé selon l'une au moins des revendications 1 à 10, caractérisé en ce que la réaction s'effectue à une température de 15 à 50°C.

12. Plasmide hybride pGMK921 tel que déposé dans *Pseudomonas putida* PpS81 (DSM 6776) et *Pseudomonas putida* GP012 (DSM 6775).
